# EUROPÄISCHE PATENTSCHRIFT

(11) **EP 0 586 915 B1**
(45) Veröffentlichungstag und Bekanntmachung des Hinweises auf die Patenterteilung: **25.04.2001**
(21) Anmeldenummer: 93112914.2
(22) Anmeldetag: 12.08.1993
(51) Int. Cl.: G01N 33/566, G01N 33/68, G01N 33/569, G01N 33/74, G01N 33/543

(54) **Verfahren zum Nachweis und zur Bestimmung von Mediatoren**
Method for detection and determination of mediators
Procédé pour la détection et la détermination des médiateurs

(30) Priorität: 29.08.1992 DE 4228839
(43) Veröffentlichungstag der Anmeldung: 16.03.1994
(73) Patentinhaber: Dade Behring Marburg GmbH, 35001 Marburg (DE)
(72) Erfinder: Enssle, Karlheinz, D-35041 Marburg-Michelbach (DE); Kurrle, Roland, D-35096 Niederweimar (DE); Langner, Klaus-Dieter, D-35041 Marburg (DE); Lauffer, Leander, D-35075 Gladenbach (DE); Pauly, Josef-Urban, D-35085 Ebsdorfergrund 8 (DE); Seiler, Friedrich-Robert, D-35041 Marburg (DE)

(56) Entgegenhaltungen:
- EP-A- 0 202 975
- EP-A- 0 367 566
- EP-A- 0 464 533
- EP-A- 0 548 867
- US-A- 5 229 501
- BIOCHEMICAL AND BIOPHYSICAL RESEARCH COMMUNICATIONS Bd. 154, Nr. 1 , 15. Juli 1988 , DULUTH, MINNESOTA US Seiten 372 - 379 MAY C. MIEDEL ET AL. 'Structural Analysis of Recombinant Soluble Human Interleukin-2 Receptor'
- THE JOURNAL OF IMMUNOLOGY. Bd. 144, Nr. 8 , 15. April 1990 , BALTIMORE US Seiten 3028 - 3033 CHARLES R. MALISZEWSKI ET AL. 'Cytokine Receptors and B Cell Functions I. Recombinant Soluble Receptors Specifically Inhibit IL-1 and IL-4-Induced B Cell Activities In Vitro'
- IMMUNOLOGY TODAY Bd. 12, Nr. 8 , August 1991 , CAMBRIDGE GB Seiten 256 - 257 SERGIO ROMAGNANI 'Human TH1 and TH2 subsets: doubt no more'

## Beschreibung

Die Erfindung betrifft ein Verfahren zum Nachweis und zur Bestimmung von Mediatoren und/oder ihren Derivaten.

Mediatoren sind z. B. Interleukine wie IL-1, TNF, IL-8 oder IL-4, Cytokine wie GM-CSF, G-CSF oder Meg-CSF oder auch Erythropoietin. Mediatoren sind wichtige Signalproteine, die von bestimmten Körperzellen, wie Lymphozyten, abgegeben werden und auf dieselben oder auf andere Körperzellen regulierend wirken können. Diese regulierende Funktion können solche Mediatoren in sehr geringer Konzentration ausüben. Im Krankheitsfalle kann das natürliche Zusammenspiel unterschiedlicher Mediatoren gestört sein. Mediatoren können dann, bezogen auf Konzentrationen dieser Mediatoren in durchschnittlichen Normalpersonen, höher oder niedriger liegen. Es ist diagnostisch wichtig, die Konzentration bestimmter Mediatoren in physiologischen Körperflüssigkeiten oder anderen Quellen wie Organhomogenaten zu bestimmen. Zudem können Personen zum Zweck der Therapie oder Prophylaxe mit bestimmten Mediatoren behandelt werden. Auch hier ist es diagnostisch von hohem Interesse, die Konzentration des applizierten Mediators oder aber anderer Mediatoren, die durch den Einfluß des applizierten Mediators in ihrer Konzentration verändert werden, zu bestimmen. So kann beispielsweise das Auftreten von IL-4 im Körper bei allergischen Erkrankungen oder Infektionen pathologisch gegenüber Normalpersonen verändert sein. Ursache kann das bei solchen Krankheiten oft erhöhte Auftreten von T-Lymphozyten der Subpopulation TH2 sein, die gegenüber der Subpopulation TH1 bevorzugt Interleukin-4 bildet (S. Romagnani, Immunology Today, Vol. 12, No. 8, 256 - 257, 1991; Else and Grencis, Parasitology Today, Vol. 7, No. 11, 313-316, 1991). Es ist außerdem diagnostisch wichtig, Mediatoren in Überständen von Zellkulturen von Lymphozyten oder anderen Körperzellen zu bestimmen.

Mediatoren, wie die oben angeführten, üben ihre positive oder negative Wirkung über membranständige Rezeptoren aus. Diese Rezeptoren binden über eine definierte Bindungsstelle an ein definiertes Epitop der Mediatoren. Anschließend erfolgt eine Signaltransduktion über den Rezeptor und/oder assoziierte Moleküle in die Zelle, in der daraufhin ein biologischer Effekt erfolgt. Die biologische Wirkung eines solchen Mediators ist also streng an die optimale Bindung an die Bindungsstelle auf dem membranständigen Rezeptor gebunden. Beispielsweise lösen Substanzen oder Mediatoren, die nicht direkt an die Bindungsstelle, sondern an andere Epitope auf dem Rezeptor binden, keine Signaltransduktion aus.

Überraschenderweise wurde nun gefunden, daß sich auch rekombinante, lösliche Rezeptoren von solchen Mediatoren zum Nachweis der Anwesenheit und der Konzentration von Mediatoren in Flüssigkeiten verwenden lassen.

Gegenstand der Erfindung ist daher ein Verfahren zum Nachweis und zur Bestimmung eines biologisch aktiven Mediators und/oder eines Derivats davon in einer Flüssigkeit, worin ein rekombinanter, löslicher Rezeptor des nachzuweisenden Mediators mit einer Probe, die den Mediator enthalten kann, in Kontakt gebracht und der an den Rezeptor gebundene Mediator durch einen für den Mediator spezifischen Antikörper direkt oder indirekt nachgewiesen wird, und worin der Mediator über die natürliche Rezeptorbindungsstelle an der Rezeptor gebunden wird.

Vorteilhaft ist dabei ein solches Verfahren, bei dem Dimere oder Multimere des Mediators oder Derivate davon bestimmt werden.

Vorteilhaft ist ferner ein solches Verfahren, bei dem der Rezeptor oder Derivate davon an eine feste Phase gebunden wird.

Vorteilhaft ist auch ein solches Verfahren, bei dem ein rekombinantes Fusionsprotein von Rezeptor und dem Fc-Teil von Antikörpern oder Derivate davon über Fc-spezifische Antikörper an die feste Phase gebunden wird.

Besonders vorteilhaft ist ein solches Verfahren, bei dem der Rezeptor oder Derivate davon über spezifische Antikörper an die feste Phase gebunden wird, anschließend Probenmaterial mit dem korrespondierenden, zu bestimmenden Mediator aufgetragen wird und der Mediator mit einem markierten Antikörper oder Antiserum mit Spezifität für den Mediator nachgewiesen wird.

Ganz besonders vorteilhaft ist ein solches Verfahren, bei dem ein rekombinantes Fusionsprotein von Rezeptor und dem Fc-Teil von Antikörpern oder Derivate davon über Fc-spezifische Antikörper an die feste Phase gebunden wird, anschließend Probenmaterial mit dem korrespondierenden zu bestimmenden Mediator oder Derivaten davon aufgetragen wird und der gebundene Mediator mit markiertem Antikörper oder Antiserum mit Spezifität für den Mediator nachgewiesen wird.

Vorteilhaft ist auch ein solches Verfahren, bei dem Konjugate von Rezeptor oder Derivate davon, gekoppelt an Substanzen, die zum Nachweis in geeigneten Meßsystemen geeignet sind, dazu verwendet werden, Mediator und/oder Derivate davon nachzuweisen, der an eine feste Phase, zum Beispiel über spezifische Antikörper, gebunden ist.

Gegenstand der Erfindung ist ferner die Verwendung eines solchen Verfahrens für ein Screening nach Agonisten oder Antagonisten des Mediators oder des Rezeptors.

Gegenstand der Erfindung ist auch die Verwendung eines solchen Verfahrens zur Bestimmung der Affinität zwischen Mediator und dessen Rezeptor.

Gegenstand der Erfindung ist ebenfalls die Verwendung eines solchen Verfahrens zur Identifizierung und Analyse von modifizierten Mediatoren ("Muteine") oder Teilen der Mediatoren (zum Beispiel Oligopeptide).

Vorteilhaft ist dabei die Verwendung des Verfahrens zur Identifizierung und Analyse von Substanzen, die die Interaktion von pathogenen Organismen (zum Beispiel Viren oder Bakterien) mit deren zellulären Rezeptoren beeinflussen. Besonders vorteilhaft ist die Verwendung eines solchen Verfahrens zur Identifizierung von Substanzen, die die Interaktion von zellulären Adhäsionsmolekülen beeinflussen.

Besonders vorteilhaft ist ein solches Verfahren, in dem der Rezeptor ein Cytokinrezeptor, ein Wachstumshormonrezeptor, ein Hormonrezeptor, ein Neurotransmitter-Rezeptor oder ein zellulärer Rezeptor für einen pathogenen Organismus, z. B. einen Virus, ist.

Ganz besonders vorteilhaft ist ein solches Verfahren, in dem der Rezeptor der Interleukin-4-Rezeptor oder ein
Derivat davon, ein Erythropoietin-Rezeptor oder ein
Derivat davon, der Interleukin 1-Rezeptor oder ein
Derivat davon, der Interleukin 7-Rezeptor oder ein
Derivat davon, der GM-CSF-Rezeptor oder ein
Derivat davon oder der IL-8-Rezeptor oder ein TNF-Rezeptor oder ein
Derivat davon ist.

Gegenstand der Erfindung ist auch ein solches Verfahren zum diagnostischen Nachweis von Interleukin-4 bei Erkrankungen mit verstärktem Auftreten von TH2-T-Zellen, zum Beispiel allergischen Erkrankungen und Infektionen.

Die Verwendung der natürlichen, biologisch wichtigen Rezeptorbindungsstelle für den Nachweis des korrespondierenden Mediators ist wesentlich, da somit ausschließlich Mediatormoleküle erfaßt werden, die auch an den natürlichen, membranständigen Rezeptor binden würden und somit biologisch aktiv sind.

Im Sinne dieser Erfindung werden unter Rezeptoren auch alle Varianten und Derivate verstanden, die in der Lage sind, den entsprechenden Mediator spezifisch zu binden.

Der Nachweis kann dadurch durchgeführt werden, daß unter geeigneten Bedingungen der rekombinante lösliche Rezeptor an die feste Phase des Nachweissystems, zum Beispiel den Kunststoff einer Mikrotitrationsplatte, gebunden wird. Solche feste Phasen sind dem Fachmann an sich bekannt. Vorteilhaft sind als feste Phasen: magnetische Partikel, Partikel aus natürlichen oder synthetischen Polymeren, z. B. sogenannte Latexpartikel oder Ionenaustauscher-Harze oder synthetische Polymere in Form von konkaven oder konvexen Artikeln, wie z. B. Mikrotitrationsplatten oder Kugeln.

Besonders vorteilhaft sind magnetisierbare Partikel, Latexpartikel oder Mikrotitrationsplatten. Ganz besonders vorteilhaft sind Mikrotitrationsplatten. Obwohl der Rezeptor nicht in membranständiger Form vorliegt, bindet er überraschenderweise mit der Affinität des natürlichen, membrangebundenen Rezeptors das entsprechende Epitop auf dem korrespondierenden Mediator. Der gebundene Mediator läßt sich nun mit dem Fachmann an sich bekannten Methoden nachweisen. Dabei kann man den gebundenen Mediator mit einem oder mehreren monoklonalen Antikörpern, die gegen ein weiteres Epitop des Mediators gerichtet sind oder durch ein Antiserum, das gegen mehrere weitere Epitope auf dem Mediator gerichtet ist, zur Reaktion bringen.

Um gebundenes Antiserum oder einen gebundenen, monoklonalen Antikörper nachzuweisen, kann dieser direkt mit entsprechenden Substanzen oder Proteinen gekoppelt werden. Solche signalgebenden Komponenten sind dem Fachmann als solche bekannt, es sind Substanzen oder Proteine, die einen quantitativen Nachweis, z. B. mittels radioaktiver Nuklide erlauben oder die eine enzymatisch katalysierte Farbreaktion ermöglichen, z. B. mittels gekoppelter Peroxidase oder die die enzymatisch katalysierte Erzeugung von Substanzen erlauben, die den Nachweis durch Lumineszenz oder Fluoreszenz ermöglichen. Vorteilhaft ist die Verwendung eines Lumineszenzlabels, besonders vorteilhaft ist dabei die Verwendung der in der EP 0 257 541 und EP 0 330 050 beschriebenen Verbindungen.

Das Antiserum oder der monoklonale Antikörper kann auch an magnetisierbare Partikel gekoppelt sein.

Zum Nachweis mit radioaktivem Nuklid kann z. B. die Methode von Siekierka, J.J. and De Gudicibus, S., Anal. Biochem. Vol. 172 (1988), 514-517 verwendet werden. Für den Nachweis durch Farbreaktion kann das Antiserum oder ein oder mehrere monoklonale Antikörper auch biotinyliert werden (King und Catino, Anal. Biochem. Vol. 188 (1990), 97-100). Das Biotin wiederum kann durch Avidin oder auch anti-Biotin nachgewiesen werden, an das Substanzen oder Proteine gekoppelt sind, die die oben aufgeführten Nachweismethoden erlauben. Zum Beispiel kann dies ein Konjugat zwischen Avidin und Peroxidase sein.

Der Nachweis von Mediatoren mit korrespondierenden rekombinanten löslichen Rezeptoren kann auch erfolgen, indem Varianten des Rezeptors verwendet werden, die über spezifisch bindende Proteine und indirekt an die Festphase, z. B. eine Mikrotitrationsplatte, gebunden werden. Eine solche Variante kann ein rekombinantes Fusionsprotein sein zwischen der extrazellulären Domäne des Rezeptors oder Teilen davon und dem Fc-Teil oder Teilen davon, von Immunglobulinen, zum Beispiel IgG-Fc derselben Species oder von anderen Species. Hier kann zunächst ein oder mehrere monoklonale Antikörper oder ein Antiserum, die mit dem Fc-Teil des Fusionsproteins spezifisch reagieren, an die feste Phase, z. B. eine Mikrotitrationsplatte, gebunden werden. Dieser monoklonale Antikörper oder dieses Antiserum binden dann das Rezeptor-Fusionsprotein spezifisch. Dies hat den Vorteil eines besseren Erhalts der Konformation des Rezeptors.

Wird zudem statt des homologen Fc-Teils zur rekombinanten Fusion mit dem Rezeptor ein Fc-Teil oder Teile von einer anderen Spezies verwendet, so sind auch Kreuzreaktionen bei Nachweis von Mediator aus physiologischen Körperflüssigkeiten, die auch Fc-tragende Proteine aufweisen können, ausgeschlossen.

Der Nachweis von Mediatoren mit korrespondierenden rekombinanten löslichen Rezeptoren kann auch erfolgen, indem zunächst an die feste Phase, z. B. an eine Mikrotitrationsplatte, ein oder mehrere monoklonale Antikörper oder ein Antiserum gebunden werden, die mit anderen oder mehreren anderen Epitope auf dem Mediator reagieren, als der korrespondierende Rezeptor. Anschließend wird der Mediator an die spezifischen Antikörper der Festphase gebunden. An den gebundenen Mediator wird nun der korrespondierende Rezeptor gebunden. An den Rezeptor können Substanzen oder Proteine gekoppelt sein, die, wie oben ausgeführt, einen quantitativen Nachweis erlauben.

Werden, wie oben ausgeführt, Fc-Fusionsproteine des Rezeptors verwendet, so können die Rezeptorproteine durch geeignete Fc-spezifische Substanzen oder Proteine nachgewiesen werden, die entweder eine direkte quantitative Bestimmung erlauben, oder an die wiederum Substanzen oder Proteine gekoppelt sind, die eine solche quantitative Messung erlauben. Beispielsweise kann der Fc-Teil oder Teile des rekombinanten Fusionsproteins durch biotinylierte monoklonale Antikörper oder Antiseren spezifisch nachgewiesen werden, die selbst durch ein Avidin-Peroxidase Konjugat detektiert werden können. Ein solcher Fc- spezifischer Nachweis könnte zum Beispiel mit einem biotinylierten monoklonalen Antikörper gegen die CH2-Domäne des Fc-Teils erfolgen. Wird zudem statt des homologen Fc-Teils zur rekombinanten Fusion mit dem Rezeptor ein Fc-Teil oder Teile davon von einer anderen Spezies verwendet, so sind auch Kreuzreaktionen beim Nachweis von Mediator aus physiologischen Körperflüssigkeiten, die auch Fc-tragende Proteine aufweisen, ausgeschlossen.

Rekombinante Rezeptoren werden auch zum Nachweis von biologisch aktiven Dimeren oder Multimeren des korrespondierenden Mediators benutzt. Z. B. liegt der Tumor-Nekrose-Faktor Alpha unter physiologischen Bedingungen als Trimer vor. Dimere oder Trimere von Mediatoren können auch synthetisch oder rekombinant hergestellt werden. Der Nachweis solcher Dimere oder Oligomere wird geführt, indem rekombinante Rezeptoren in einem Assay vom Sandwich-Typ sowohl zur Bindung des Dimers oder Oligomers an die feste Phase, als auch zum Nachweis von daran gebundenem Dimer oder Oligomer über die freibleibenden Epitope genutzt wird. Der Nachweis kann auch geführt werden, indem mit unterschiedlichen Nachweissubstanzen markierte Rezeptoren oder Varianten davon genutzt werden.

Der Nachweis von Dimeren oder Oligomeren kann auch erfolgen, indem ein Epitop bei Dimeren oder ein oder mehrere Epitope bei Oligomeren durch entsprechend markierten oder an die feste Phase gebundenen Rezeptor gebunden wird und die freibleibenden Epitope durch monoklonale Antikörper nachgewiesen werden, die die biologische Wirkung des entsprechenden Mediators neutralisieren und zum Nachweis in geeigneter Weise markiert sind. Beispielsweise können MTP mit 100 µl pro Vertiefung eines monoklonalen bzw. polyklonalen anti-human IgG-Antikörpers in PBS mit eine Konzentration von 5 µg/ml beschichtet werden. Nach einer Inkubationszeit von 24 h bei Raumtemperatur, können die MTP mehrmals mit einer Puffer-lösung gewaschen, getrocknet und einzeln luftdicht mit einem Trocknungsmittel verpackt werden. Diese gebrauchsfertigen MTP können über einen längeren Zeitraum gelagert werden.

Zum Nachweis von EPO-Dimer werden diese Platten vor Beginn der Testdurchführung zweimal mit einer Waschlösung (WP, bestehend aus PBS mit 0.05 % Tween 20) gewaschen. 100 µl einer EPO-R-Fc-Lösung (100 ng/ml in WP) werden dann in alle Vertiefungen dispensiert und die Platten für 30 min bei 37 °C inkubiert. Eventuell verbliebene, freie Fc-Bindungsstellen werden danach mit 10 % Humanserum (Behringwerke Marburg, FRG) abgeblockt. Nach mehrmaligem Waschen werden anschließend je 100 µl pro Vertiefung einer seriellen Verdünnungsreihe von bioaktivem EPO-Dimer (rekombinantem humanem EPO-Fc-Fusionsprotein) pipettiert. Die Bindung des EPO-Dimers in den Proben erfolgt dann während 2 h bei Raumtemperatur. Nach mehrmaligem Waschen mit WP wird das gebundene EPO-Fc durch die Zugabe eines EPO-Rezeptor-Enzym-Konjugates detektiert. Hierzu werden je 100 µl/Vertiefung eines EPO-Rezeptor-Meerrettichperoxidase-Konjugates (0.5 µg/ml in WP + 2 % Rinderalbumin) dispensiert. Nach einer Inkubation von 1 h bei Raumtemperatur werden die MTP mehrmals gewaschen. Ein Signal wird anschließend durch die Oxidation von o-Phenylendiamin mittels der immobilisierten Peroxidase generiert.

Die oben aufgeführten Möglichkeiten des Nachweises von Mediatoren mit Hilfe von rekombinanten Rezeptoren oder Varianten davon können in vielfältiger Weise angewendet werden.

Natürliche oder rekombinante Mediatoren können nachgewiesen werden. Die Mediatoren können gereinigt, in geeigneter Testflüssigkeit verdünnt oder aus natürlichen Körperflüssigkeiten wie Blutplasma, Blutseren, Vollblut, Urin, Stuhlproben, Augenflüssigkeit, Magenflüssigkeit, Liquor, Sputum oder in geeignet aufbereiteten Organhomogenaten oder in Überständen von Zellkulturen von Körperzellen in geeignet aufbereiteter Form nachgewiesen werden.

Die Nachweismethoden können des weiteren genutzt werden:
- um ein Rezeptorscreening zu betreiben, d.h. um Agonisten oder Antagonisten zu finden, die die Interaktion zwischen Mediator und dessen korrespondierendem Rezeptor inhibieren oder fördern;
- um die Bindungsaktivität von Rezeptor oder Ligand zu bestimmen;
- zur Identifizierung und Analyse von modifizierten Mediatoren ("Muteinen") oder Teilen der Mediatoren (z. B. Oligopeptiden);
- zur Identifizierung und Analyse von Substanzen, die die Interaktion von pathogenen Organismen (z. B. Viren oder Bakterien) mit deren zellulären Rezeptoren beeinflussen;
- zur Identifizierung von Substanzen, die die Interaktion von zellulären Adhäsionsmolekülen beeinflussen.

Die folgenden Beispiele dienen der Erläuterung der Erfindung, schränken diese aber nicht ein.

### Verwendete Abkürzungen:

- BHK: Baby Hamster Kidney-Zellen
- BSA: Bovines Serumalbumin
- EPO: Erythropoietin
- EPOR-Fc: Erythropoietin-Rezeptor Fusionsprotein mit humanem IgG1-Fc (rekombinant, löslich)
- G-CSF: Granulozyten-Kolonie-Stimulierender-Faktor
- GM-CSF: Granulozyten-Makrophagen Kolonie-Stimulierender-Faktor
- IL-1: Interleukin-1
- IL-2: Interleukin-2
- IL-4: Interleukin-4
- IL-4R: Interleukin-4 Rezeptor (rekombinant, löslich)
- IL-4R/Fc: Interleukin-4 Rezeptor Fusionsprotein mit dem Fc-Teil eines Immunglobulins (rekombinant, löslich)
- IL-6: Interleukin-6
- IL-7: Interleukin-7
- Meg-CSF: Megakaryozyten-Kolonie-Stimulierender-Faktor
- MTP: Mikrotitrationsplatten
- PBS: Phosphate Buffered Saline
- PCR: Polymerase-Kettenreaktion
- POD: Peroxidase
- TH1: T-Helfer Lymphozyten des Typs 1
- TH2: T-Helfer Lymphozyten des Typs 2
- TMB: Tetramethylbenzidine
- TNF: Tumor Nekrose Faktor
- WPA: Waschlösung A
- WPB: Waschlösung B

Die Herstellung des IL-4R kann beispielsweise nach der internationalen Patentanmeldung WO 90/05183 erfolgen.

### Beispiel 1:

### Immunorezeptor Assayverfahren zur Bestimmung der Anwesenheit und der Konzentration von humanem IL-4 in Flüssigkeitsproben

Für die Beispiele 1 und 2 wurden die in Idzerda et al. (1990, J. Exp. Med. 171, 861-873) bzw. Maliszewski et al. (1990, J. Immunol. 144, 30283033) definierten extrazellulären Regionen bzw. natürlicherweise vorkommenden löslichen Formen von humanem bzw. murinem IL-4-Rezeptor (im folgenden als huIL-4R bzw. muIL-4R bezeichnet) verwendet, die nach Doppelselektion mit Methotrexat und G418 (EP-A 0330 977) von stabil exprimierenden BHK-Zellen als lösliches Protein in das Kulturmedium sezerniert und über Immuno-Affinitätschromatographie gereinigt wurden. Darüberhinaus wurden Rezeptor/ Immunglobulin-Fusionsproteine (EP-A1-0 464 533) verwendet, die aus der extrazellulären Region von humanem bzw. murinem IL-4-Rezeptor mit Hinge-, CH2- und CH3-Domänen eines humanen IgGl- bzw. murinen IgG2b-Moleküls (Zettlmeißl et al. (1990) DNA and Cell Biol. 9, 347-353) bestehen (im folgenden als huIL-4R/Fc bzw. muIL-4R/Fc bezeichnet) und ebenfalls nach Expression in BHK-Zellen über Protein A-Sepharose-Affinitätschromatographie gereinigt wurden.

MTP wurden 16 h mit 5 µg/ml rekombinantem, humanem IL-4R oder IL-4R/Fc PBS mit einem Volumen von 100 µl/Testvertiefung bei 4 °C beschichtet. Anschließend wurden die Platten dreimal mit PBS mit 0,05 % (w/v) Tween-20 und mit 0,1 % (w/v) BSA (im folgenden als Waschpuffer (WPA) bezeichnet) gewaschen, um nichtgebundenes Beschichtungsmaterial zu entfernen. Anschließend wurden die Platten bei 37 °C mit 200 µl Puffervolumen pro Testvertiefung 2 h mit PBS mit 0,05 % (w/v) Tween-20 und 2 % (w/v) BSA inkubiert, um freie Bindungsstellen abzublocken. Nun wurde humanes, rekombinantes IL-4 (Immunex, Seattle, USA, Lot 1898-022) in WPA auf die angegebenen Testkonzentrationen verdünnt und aufgetragen. Es folgte eine Inkubation der Testplatten für 90 min bei 37 °C. Anschließend wurden die Testplatten dreimal wie oben angegeben mit WPA gewaschen um nichtgebundenes IL-4 zu entfernen. Zum Nachweis von gebundenem IL-4 wurde nun ein Antiserum vom Kaninchen mit Spezifität für humanes IL-4 (BL4P) (Genzyme, Cambridge, USA) in einer Konzentration von 2 µg/ml in WPA verdünnt und mit einem Volumen von 100 µl/ Testvertiefung zugegeben. Es folgte eine Inkubation von 90 min bei 37 °C. Danach wurde nichtgebundenes Antiserum durch dreimaliges Waschen mit WPA entfernt. Zum Nachweis des gebundenen Antiserums wurde biotinyliertes Antiserum von der Ziege mit Spezifität für Kaninchen-Immunglobulin (A0207, Vector Inc., Burlingame, USA) in einer Konzentration von 0,15 µg/ml verdünnt, in WPA in 100 µl/Kulturvertiefung zugegeben und eine h bei 37 °C inkubiert. Um nichtgebundenes Antiserum zu entfernen wurde nun mit WPA dreimal gewaschen. Anschließend wurde Streptavidin-POD (RPN 1213,44B, Amersham, UK) in einer Testverdünnung von 1:2000 in WPA verdünnt in 100 µl Volumen/Testvertiefung zugegeben und eine h bei 37 °C inkubiert. Zum enzymatischen Nachweis des gebundenen Streptavidin-POD-Konjugats und damit indirekt des gebundenen IL-4 wurde das Chromogen TMB (OUVF 925, Behringwerke, Marburg, FRG) 1:10 mit Behring-Substratpuffer (Behringwerke Marburg, FRG, OUVG 945) verdünnt und mit 100 µl/Kulturvertiefung auf die Testplatten gegeben. Nun wurde 1 h bei Raumtemperatur inkubiert. Anschließend wurde die Reaktion mit 0,5 N Schwefelsäure mit 100 µl/Kulturvertiefung Kulturvertiefungen abgestoppt und die Extinktion der individuellen bei 450 um, Referenzwellenlänge 650 nm, gemessen.

Angegeben sind jeweils die Mittelwerte von Doppelbestimmungen mit Variationskoeffizient (VK in %). Die Ergebnisse sind in Tabelle 1A dargestellt. Bei gleicher Beschichtungskonzentration wurde mit huIL-4R/Fc gegenüber huIL-4R ein etwas höherer Background und eine insgesamt höher liegende Extinktion beobachtet. Dies wirkt sich aber für die Nachweisempfindlichkeit für humanes IL-4 nicht aus. Die Reaktion war hochspezifisch. Es erfolgte keine Reaktion, wenn statt humanem IL-4 murines IL-4 eingesetzt wurde. Auch andere Mediatoren, z. B. humanes IL-1-α, IL-1-β, humanes TNF-α, humanes TNF-β, humanes IL-2, humanes IL-6, humanes IL-7 zeigten keine Reaktion. Diese Kontrollen wurden mit IL-4R/Fc als Beschichtungsmaterial durchgeführt und sind in Tabelle 1B dargestellt. Angegeben sind jeweils die Mittelwerte von Doppelbestimmungen mit Variationskoeffizient (VK in %). Bei den Kontrollen wurden die Extinktionen anhand der Standard kurve in Konzentration (pg/ml) umgerechnet, lagen aber jeweils unter Background. Der Nachweis kann auch durchgeführt werden, wenn humanes IL-4 beispielsweise in Kulturmedium oder Serum vorliegt.

### Beispiel 2:

### Immunorezeptor Assayverfahren zur Bestimmung der Anwesenheit und der Konzentration von murinem IL-4 in Flüssigkeitsproben

Die Herstellung des murinen IL-4R/Fc wurde durchgeführt, wie in Beispiel 1 beschrieben. MTP wurden 16 h mit 5 µg/ml rekombinantem, murinen IL-4R/Fc in PBS mit einem Volumen von 100 µl/Testvertiefung bei 4 °C beschichtet. Anschließend wurden die Platten dreimal mit PBS mit 0,05 % (w/v) Tween-20 und mit 0,1 % (w/v) BSA (im folgenden als WPA bezeichnet) gewaschen, um nichtgebundenes Beschichtungsmaterial zu entfernen. Dann wurden die Platten bei 37 °C mit 200 µl Puffervolumen pro Testvertiefung 2 h mit PBS mit 0,05 % (w/v) Tween-20 und 2 % (w/v) BSA inkubiert, um freie Bindungsstellen abzublocken. Nun wurde murines rekombinantes IL-4 (Lot 2871-023, Immunex, Seattle, USA) in WPA auf die angegebenen Testkonzentrationen verdünnt und aufgetragen. Es folgte eine Inkubation der Testplatten für 90 min bei 37 °C. Anschließend wurden die Testplatten dreimal wie oben angegeben mit WPA gewaschen, um nichtgebundenes IL-4 zu entfernen. Zum Nachweis von gebundenem Il-4 wurde nun ein monoklonaler Antikörper von der Ratte mit Spezifität für murines IL-4 (MM450 D Lot 103023, Endogen, Boston, USA) in einer Konzentration von 2 µg/ml in WPA verdünnt und mit einem Volumen von 100 µl/Testvertiefung zugegeben. Es folgte eine Inkubation von 90 min bei 37 °C. Danach wurde nicht-gebundener monoklonaler Antikörper durch dreimaliges Waschen mit WPA entfernt. Zum Nachweis des gebundenen Antikörpers wurde jetzt biotinyliertes Antiserum von der Ziege mit Spezifität für Ratte-Immunglobulin (3010-08 Lot B022 N222, Southern Biotechnology Assoc., Birmingham, USA) in einer Konzentration von 0,25 µg/ml verdünnt in WPA in 100 µl/Kulturvertiefung zugegeben und 1 h bei 37 °C inkubiert. Um nicht gebundenes Antiserum zu entfernen wurde nun mit WPA dreimal gewaschen. Anschließend wurde Streptavidin-POD (RPN 1213,44 B, Amersham, UK) in einer Testverdünnung von 1:2000 in WPA verdünnt in 100 µl Volumen/Testvertiefung zugegeben und 1 h bei 37 °C inkubiert. Zum enzymatischen Nachweis des gebundenen Streptavidin-POD-Konjugats und damit des gebundenen IL-4 wurde das Chromogen TMB (Behringwerke Marburg, FRG, OUVF 925) 1:10 mit Behring-Substratpuffer (Behringwerke Marburg, FRG, OUVG 945) verdünnt und mit 100 µl/Kulturvertiefung auf die Testplatten gegeben. Nun wurde eine h bei Raumtemperatur inkubiert. Anschließend wurde die Reaktion mit 0,5 N Schwefelsäure mit 100 µl/Kulturvertiefung abgestoppt und die Extinktion der individuellen Kulturvertiefungen bei 450 nm, Referenzwellenlänge 650 nm gemessen. Die Ergebnisse sind in Tabelle 2A dargestellt. Die Reaktion war hochspezifisch. Es erfolgte keine Reaktion, wenn statt murinem IL-4 humanes IL-4 oder andere Mediatoren, z. B. murines IL-1-α, murines IL-1-β oder murines TNF-α verwendet wird. Diese Kontrollen sind in Tabelle 2B dargestellt. Angegeben sind jeweils die Mittelwerte von Doppelbestimmungen mit Variationskoeffizient (VK in %). Bei den Kontrollen wurden die Extinktionen anhand der Standardkurve in Konzentration (pg/ml) umgerechnet, lagen aber jeweils unter Background. Der Nachweis kann auch durchgeführt werden, wenn murines IL-4 beispielsweise in Kulturmedium oder Serum vorliegt.

### Beispiel 3:

### Immunorezeptor Assayverfahren zur Bestimmung der Anwesenheit und der Konzentration von bioaktivem EPO in Flüssigkeitsproben mit einer Kombination bestehend aus Festphasen-gebundenem anti-EPO Antikörper (monoklonal oder poly-klonal) und einem EPO-Rezeptor-Enzymkonjugat

Die cDNA für den menschlichen EPO-Rezeptor ist kürzlich isoliert worden (Winkelmann et al., (1990), Blood 76, 24-30). Zwei Oligonukleotide wurden synthetisiert, die mit Sequenzen in der 5'-untranslatierten Region (A: 5' AGG CAG CTG CTG ACC AAG CTT TGG ACT GTG CCG GGG GC 3') bzw. kodierenden Region (B: 5' AGA GCC TCA GGA TGA GGG GAT CCA GGT CGC TAG CGC 3') der EPO-Rezeptor-cDNA hybridisieren können. Oligonukleotid A ist dabei teilweise homolog zur Sequenz des kodierenden Stranges und beinhaltet eine HindIII-Schnittstelle, Oligonukleotid B ist teilweise homolog zum nicht-kodierenden Strang und beinhaltet eine BamHI-Schnittstelle. PCR an der EPO-Rezeptor-cDNA mittels der beiden Oligonukleotide A und B ergibt ein DNA-Fragment, das die gesamte kodierende Sequenz für die extrazelluläre Region des EPO-Rezeptors enthält. Das Leseraster in der durch Oligonukleotid B eingeführten BamHI-Schnittstelle ist dabei dergestalt, daß die Nukleotidfolge GAT als Asparaginsäure translatiert wird. Das PCR-Fragment wurde mit HindIII und BamHI behandelt und in den mit HindIII und BamHI geöffneten pCD4E gamma 1H-Vektorbackbone einligiert. Das resultierende Plasma erhielt die Bezeichnung pEPORFc. pCD4E gamma 1H ist der aus der EP 0 325 262 A2 bekannte Vektor pCD4E gamma 1, bei dem die stromabwärts der singulären BamHI-Schnittstelle befindliche HindIII-Schnittstelle durch partielle Spaltung mit HindIII, Auffüllen der HindIII-Überhänge mittels Klenow-Enzym und Religierung deletiert wurde. pEPORFc kodiert für das Protein EPORFc bestehend aus der extrazellulären Region des humanen EPO-Rezeptors fusioniert an Hinge-, CH2- und CH3-Domäne eines humanen IgG1-Moleküls (EP 0 464 533 A1). pEPORFc wurde in BHK-Zellen transfiziert und stabile Klone wurden nach Doppelselektion mit Methotrexat und G418 erhalten (EP 0 330 977). Typische Expressionsraten lagen bei 5 µg/ml Überstand, aus dem EPORFc mittels Chromatographie auf Protein A-Sepharose isoliert wurde (EP 0 464 533 A1).

MTP wurden mit 100 µl pro Vertiefung beschichtet, enthaltend eine Konzentration von 5 µg/ml eines monoklonalen bzw. polyklonalen anti-EPO-Antikörpers in isotonem PBS, der ein von der Rezeptorbindungsstelle unterschiedliches und unabhängiges Epitop erkennt. Nach einer Inkubationszeit von 24 h bei Raumtemperatur wurden die MTP mehrmals mit 0,05 m TRIS/Citrat gewaschen, getrocknet und einzeln luftdicht mit einem Trocknungsmittel verpackt. Diese gebrauchsfertigen MTP können über einen längeren Zeitraum gelagert werden. Vor Beginn des Assays wurden die Platten zweimal mit einer WPB (bestehend aus PBS mit 0.05 % Tween 20) gewaschen. Anschließend werden je 100 µl/Vertiefung einer seriellen Verdünnungsreihe von rekombinantem humanem EPO pipettiert. Die Bindung des EPOs in den Proben erfolgt während 2 h bei Raumtemperatur. Nach mehrmaligem Waschen mit WPB wurde das gebundene EPO durch die Zugabe des EPO-Rezeptor-Enzym-Konjugates detektiert. Hierzu wurden je 100 µl/Vertiefung eines EPO-Rezeptor-Fc Meerrettichperoxidase-Konjugates (0.5 µg/ml in WPB + 2 % BSA) dispensiert. Nach einer Inkubation von 1 h bei Raumtemperatur wurden die MTP mehrmals gewaschen. Ein Signal wurde generiert durch die Oxidation von o-Phenylendiamin mittels der POD. Die Extinktion konnte anschließend bei 492 nm, Referenzwellenlänge 650 nm, gemessen werden.

Das EPO-Rezeptor-Fc-Meerettichperoxidase-Konjugat wurde folgendermaßen synthetisiert: Zunächst wurde das Rezeptor-Fc Fusionsprotein in das Maleimido-Derivat überführt nach der Methode von Tanimore et al., J.Immunol. Meth. 62, S. 123 - 131 (1983). Parallel wurde Meerrettichperoxidase (Boehringer-Mannheim, Mannheim) SH-aktiviert gemäß King et al., Biochemistry 17, S. 1499 - 1506 (1978) und zum Maleimido-EPO-Rezeptor-Fusionsprotein gegeben, wobei sich stabile Enzym-Konjugate bildeten. Tabellen 3 A - C illustrieren die Ergebnisse dieses "Sandwich type" Immunorezeptor Assays für EPO. Benutzt wurde ein EPO-Rezeptor-Fc-Fusionsprotein-Peroxidase-Konjugat zur Detektion und verschiedene an die Festphase gebundene monoklonale bzw. polyklonale Antikörper. Wie die Ergebnisse zeigen, ist diese Art von Testsystem in der Lage, die Anwesenheit und die Konzentration von bioaktivem EPO bis 2 pg/ml oder weniger zu detektieren.

### Beispiel 4:

### Immunorezeptor Assayverfahren zur Bestimmung der Anwesenheit und der Konzentration von bioaktivem EPO in Flüssigkeitsproben mit einer Kombination bestehend aus Festphasen-gebundenem anti-human IgG Antikörper, einem EPO-R-Fc-Fusionsprotein und verschiedenen EPO-AntikörperEnzymkonjugaten, die ein von der Rezeptorbindungsstelle unterschiedliches Epitop erkennen

MTP wurden mit 100 µl pro Vertiefung beschichtet, ent-haltend eine Konzentration von 5 µg/ml eines monoklonalen bzw. polyklonalen anti-human IgG-Antikörpers in PBS. Nach einer Inkubationszeit von 24 h bei Raumtemperatur, wurden die MTP mehrmals mit 0,05 M TRIS/Citrat gewaschen, ge-trocknet und einzeln luftdicht mit einem Trocknungsmittel verpackt. Diese gebrauchsfertigen MTP können über einen längeren Zeitraum gelagert werden. Vor Beginn des Assays wurden die Platten zweimal mit einer WPB (bestehend aus PBS mit 0.05 % Tween 20) gewaschen. 100 µl einer EPO-R-Fc-Lösung (100 ng/ml in WPB) wurden in alle Vertiefungen dispensiert und die Platten für 30 min bei 37 °C inkubiert. Nach mehrmaligem Waschen wurden anschließend je 100 µl/Vertiefung einer seriellen Verdünnungsreihe von rekombinantem, humanem EPO pipettiert. Die Bindung des EPOs in den Proben erfolgte während 2 h bei Raumtemperatur.

Nach mehrmaligem Waschen mit WPB wurde das gebundene EPO durch die Zugabe eines Anti-EPO-Antikörper-Enzym-Konjugates (Behringwerke, Marburg, FRG) detektiert. Hierzu wurden je 100 µl/Vertiefung eines Anti-EPO-Antikörper-Meerrettichperoxidase-Konjugates (0.5 µg/ml in WP + 2% BSA) dispensiert. Nach einer Inkubation von 1 h bei Raumtemperatur wurden die MTP mehrmals gewaschen. Ein Signal wurde generiert durch die Oxidation von o-Phenylendiamin mittels der POD.

Das Anti-EPO-Antikörper-POD-Konjugat wurde synthetisiert, wie oben für das EPO-Rezeptor-Fusionsprotein schon beschrieben.

Tabellen 4 A - D illustrieren die Ergebnisse dieses "Sandwich type" Immunorezeptor Assays für EPO. Benutzt wurde ein EPO-Rezeptor-Fc-Fusionsprotein gebunden an einen Festphasen-fixierten anti-human IgG Antikörper sowie verschiedene Anti-EPO-Antikörper-Peroxidase-Konjugate.

Wie die Ergebnisse zeigen, ist diese Art von Testsystem in der Lage, wie in Beispiel 3, die Anwesenheit und die Konzentration von bioaktivem EPO bis 2 pg/ml oder weniger zu detektieren.

### Beispiel 5:

### Immuno-Rezeptor-Assay zum Nachweis der Anwesenheit und der Konzentration von bioaktiven EPO-Dimeren und Oligomeren in Flüssigkeitsproben gemäß Beispiel 1 oder 2 unter Benutzung eines monoklonalen Antikörpers, der die Bindung des Rezeptors an EPO kompetitiv inhibieren kann

Das Beispiel wurde durchgeführt, wie unter Beispiel 4 beschrieben, lediglich mit dem Unterschied, daß nach Inkubation mit EPO-R/Fc eventuell verbliebene freie Fc- Bindungsstellen durch Inkubation mit 10 % Humanserum (Behringwerke Marburg, FRG) abgeblockt wurden und daß die verwendeten monoklonalen Antikörper (89-146-057) die Bindung des Rezeptors an Erythropoietin kompetitiv inhi-bieren können. Als aktives EPO-Dimer wurde ein Fusionsprotein aus humanem EPO und einem humanem Fc-Gamma (1)-Fragment benutzt, das bei der Biosynthese in der Zelle dimerisiert wird (EP 0 464 533 A1). Dieses Fusionsprotein wurde über eine Protein A-Affinitätschro matograpie gereinigt und ist biologisch aktiv. Zur Herstellung biologisch inaktiver Dimere/Oligomere wurde rekombinantes, humanes EPO mit Glutardialdehyd vernetzt und anschließend mittels Gelpermeationschromatographie die EPO-Dimere/Oligomere vom verbliebenen EPO-Monomer abgetrennt.

In Tabelle 5 sind die entsprechenden Daten für diesen Immunorezeptorassay zur Bestimmung der Anwesenheit und der Konzentration von biologisch aktiven EPO-Dimeren/-Oligomeren zusammengefaßt. Benutzt wurde ein EPO-Rezeptor-Fc-Fusionsprotein gebunden an einen Festphasen-fixierten anti-human IgG Antikörper sowie das Peroxidase-Konjugat des neutralisierenden Anti-EPO-Antikörper 89-146-057. Wie die Ergebnisse zeigen, kann dieses Testsystem zwischen biologisch aktiven und nichtaktiven EPO-Dimeren/Oligomeren unterscheiden und ist in der Lage, die Anwesenheit und die Konzentration von bioaktiven Dimere/Oligomeren bis auf 1 pg/ml und weniger zu detektieren.

### Beispiel 6:

### Detektion von GM-CSF mittels eines GM-CSF-Rezeptor/Fc-Fusionsproteins

Aus der DE-A-40 20 607 ist das Plasmid pGM-CSFR/Fc bekannt. Es kodiert für das Protein GM-CSFR/Fc, bestehend aus der extrazellulären Region des humanen GM-CSF-Rezeptors fusioniert an Hinge, CH2- und CH3-Domäne eines huma-en IgG1-Moleküls (EP 0 464 533 A1). pGM-CSFR/Fc wurde in BHK-Zellen transfiziert und stabile Klone wurden nach Doppelselektion mit Methotrexat und G418 erhalten (EP 0 330 977). Typische Expressionsraten lagen bei 10 µg/ml Überstand, aus dem GM-CSFR/Fc mittels Chromatographie auf Protein A-Sepharose isoliert wurde (EP 0 464 533 A1). MTP wurden mit einem Kaninchenantiserum gegen die CH2-Domäne eines menschlichen IgG1-Moleküls vorbeschichtet (DE P 4020 607.6). GM-CSFR/Fc (100 µl, 3 µg/ml in PBS enthaltend 10 % BSA) wurden an diese Festphase gebunden (1 h, Raumtemperatur). Nach dreimaligem Waschen mit PBS enthaltend 0,05 % Tween-20 (WPB) wurden eventuell verbliebene freie CH2-Bindungsstellen auf der Festphase durch Inkubation mit 20 % Humanserum in PBS ent-haltend 10 % BSA abgesättigt (100 µl, 1h, Raumtemperatur). Danach wurde mit den in Tab. 6 gezeigten Konzentrationen an rekombinantem humanem GM-CSF in PBS enthaltend 1 % BSA inkubiert (100 µl, lh, Raumtemperatur). Nach dreimaligem Waschen mit WPB erfolgte der Nachweis des gebundenen GM-CSF mit dem Meerrettichperoxidase-markierten monoklonalen anti-GM-CSF Antikörper 932/698 (Behringwerke, Marburg, FRG) in den angegebenen Verdünnungen in PBS enthaltend 1 % BSA (100 µ1, 30 min, Raumtemperatur) und anschließender Farbentwicklung in 100 µl TMB-Substratlösung (Behringwerke Marburg, FRG). Tab. 6 zeigt, daß in diesem Testaufbau abhängig von der verwendeten Antikörper-Konjugatkonzentration ein Nachweis von GM-CSF ab einer Konzentration von etwa 10 pg/ml erfolgen kann.

### Bestimmung der Anwesenheit und Konzentration von humanem Interleukin-4

**Tabelle 1A**

| Konzentration Humanes IL-4 (pg/ml) | Zur Beschichtung verwendete Proteine | | | |
|---|---|---|---|---|
| | 5 µg/ml hu IL-4R | | 5 µg/ml hu IL-4R/Fc | |
| | optische Dichte | VK (%) | optische Dichte | VK (%) |
| 200 | 3.729 | 5.4 | 4.048 | 3.6 |
| 100 | 1.532 | 0.4 | 3.631 | 2.3 |
| 50 | 0.530 | 5.4 | 1.694 | 2.6 |
| 25 | 0.241 | 5.0 | 0.817 | 4.1 |
| 12.5 | 0.130 | 7.5 | 0.465 | 2.2 |
| 6.25 | 0.102 | 12.4 | 0.340 | 4.9 |
| 3.125 | 0.095 | 28.0 | 0.306 | 11.2 |

**Tabelle 1B**

| | | |
|---|---|---|
| Kontrollen: Angegeben sind die maximal eingesetzten Konzentrationen der Proben und die aus der Extinktion anhand Tabelle 1A ermittelten Konzentrationen | | |
| hu IL-1 α | 719-27 | 7.5 ng/ml < 31 pg/ml |
| hu IL-1 β | 693-28 | 16.6 ng/ml < 31 pg/ml |
| hu-TNF α | IMMUNEX 2561-100 SKG 6-25-91 | 50 ng/ml < 31 pg/ml |
| hu-TNF α | GENZYME B1089 | 50 ng/ml < 31 pg/ml |
| hu IL-2 | ROUSSEL UCLAF 49637, Lot 22660-162 C | 5000 IU/ml < 31 pg/ml |
| hu IL-6 | Kulturüberstand 980-5-2 | 5000 IU/ml < 31 pg/ml |
| hu IL-7 | IMMUNEX | 50 ng/ml < 31 pg/ml |
| mu IL-4 | IMMUNEX | 50 ng/ml < 31 pg/ml |

### Bestimmung der Anwesenheit und Konzentration von murinem Interleukin-4

**Tabelle 2A**

| Konzentration Murines IL-4 (pg/ml) | Zur Beschichtung verwendete Proteine | |
|---|---|---|
| | 5 µg/ml mu IL-4R/Fc | |
| | optische Dichte | VK (%) |
| 2000 | 2.533 | 4.24 |
| 1000 | 1.61 | 3.95 |
| 500 | 0.849 | 5.42 |
| 250 | 0.487 | 16.84 |
| 125 | 0.292 | 2.66 |
| 62.5 | 0.191 | 11.85 |
| 31.25 | 0.156 | - |
| 0 | 0.115 | 10.5 |

**Tabelle 2B**

| | | |
|---|---|---|
| Kontrollen: Angegeben sind die maximal eingesetzten Konzentrationen der Proben und die aus der Extinktion anhand Tabelle 2A ermittelten Konzentrationen | | |
| mu IL-1 α | GENZYME B0557 | 100 IU/ml < 31 pg/ml |
| mu IL-1 β | GENZYME 1921-01 | 10 ng/ml < 31 pg/ml |
| mu-TNF α | GENZYME B0114 | 100 ng/ml < 31 pg/ml |
| hu IL-4 | IMMUNEX | 50 ng/ml < 31 pg/ml |

### Tabelle 3

### Sandwich Immuno - Erythropoietinrezeptor-Assay

Festphasen gebundene Anti-EPO-Antikörper mit verschiedenen Idiotypen. Alle Antikörper erkennen Epitope, die von der Rezeptorbindungsstelle verschieden sind. Die Detektion des gebundenen Erythropoietin erfolgte mittels eines EPO-Rezeptor-Fc-Fusionsproteins gekoppelt an Peroxidase.

**Tabelle 3A**

| [EPO] in pg/ml | Monoklonaler Antikörper 89-113-069 (optische Dichte bei 492 nm) | |
|---|---|---|
| | Mittelwert | Standardabw. |
| 1000 | 4.029 | 0.053 |
| 500 | 3.466 | 0.034 |
| 250 | 2.528 | 0.057 |
| 125 | 1.548 | 0.029 |
| 63 | 0.872 | 0.025 |
| 31 | 0.507 | 0.012 |
| 16 | 0.268 | 0.016 |
| 8 | 0.130 | 0.006 |
| 4 | 0.066 | 0.004 |
| 2 | 0.045 | 0.003 |
| 1 | 0.034 | 0.003 |
| 0 | 0.021 | 0.003 |
| Blank | 0.015 | 0.002 |

**Tabelle 3B**

| [EPO] in pg/ml | Monoklonaler Antikörper 89-113-107 (optische Dichte bei 492 nm) | |
|---|---|---|
| | Mittelwert | Standardabw. |
| 1000 | 1.831 | 0.024 |
| 500 | 1.263 | 0.027 |
| 250 | 0.626 | 0.017 |
| 125 | 0.252 | 0.009 |
| 63 | 0.113 | 0.010 |
| 31 | 0.063 | 0.007 |
| 16 | 0.044 | 0.002 |
| 8 | 0.033 | 0.002 |
| 4 | 0.026 | 0.001 |
| 2 | 0.022 | 0.002 |
| 1 | 0.021 | 0.001 |
| 0 | 0.019 | 0.001 |
| Blank | 0.016 | 0.001 |

**Tabelle 3C**

| [EPO] in pg/ml | Polyklonaler Kaninchen Anti-EPO-Antikörper (optische Dichte bei 492 nm) | |
|---|---|---|
| | Mittelwert | Standardabw. |
| 1000 | 3.053 | 0.033 |
| 500 | 2.248 | 0.022 |
| 250 | 1.153 | 0.031 |
| 125 | 0.585 | 0.019 |
| 63 | 0.296 | 0.020 |
| 31 | 0.157 | 0.007 |
| 16 | 0.094 | 0.005 |
| 8 | 0.065 | 0.004 |
| 4 | 0.046 | 0.003 |
| 2 | 0.034 | 0.003 |
| 1 | 0.026 | 0.001 |
| 0 | 0.025 | 0.002 |
| Blank | 0.014 | 0.002 |

### Tabelle 4

### Sandwich Immuno-Erythropoietinrezeptor-Bindungsassay

Festphasen gebundener Anti-human IgG monoklonaler Antikörper (5µg/ml)

Erythropoietinrezeptor-Fc-Fusionsprotein (100 n g/ml - 30 min/RT)

Die Detektion des gebundenen Erythropoietins erfolgte mittels Anti-EPO-Antikörpern gekoppelt an Meerrettichperoxidase.

**Tabelle 4A**

| [EPO] in pg/ml | Monoklonaler Antikörper 89-146-050 Peroxidase-Konjugat (optische Dichte bei 492 nm) | |
|---|---|---|
| | Mittelwert | Standardabw. |
| 1000.0 | 4.206 | 0.079 |
| 500.0 | 2.509 | 0.067 |
| 250.0 | 1.538 | 0.064 |
| 125.0 | 0.822 | 0.010 |
| 62.5 | 0.424 | 0.003 |
| 31.2 | 0.286 | 0.015 |
| 15.6 | 0.145 | 0.007 |
| 7.8 | 0.119 | 0.006 |
| 3.9 | 0.077 | 0.005 |
| 2.0 | 0.064 | 0.005 |
| 1.0 | 0.057 | 0.005 |
| 0.5 | 0.049 | 0.005 |
| 0.2 | 0.042 | 0.002 |
| 0.0 | 0.041 | 0.002 |
| Blank | 0.037 | 0.001 |

**Tabelle 4B**

| [EPO] in pg/ml | Monoklonaler Antikörper 89-113-069-Peroxidase-Konjugat (optische Dichte bei 492 nm) | |
|---|---|---|
| | Mittelwert | Standardabw. |
| 1000.0 | 3.953 | 0.061 |
| 500.0 | 3.335 | 0.100 |
| 250.0 | 2.417 | 0.065 |
| 125.0 | 1.479 | 0.045 |
| 62.5 | 0.845 | 0.031 |
| 31.2 | 0.485 | 0.022 |
| 15.6 | 0.262 | 0.026 |
| 7.8 | 0.125 | 0.010 |
| 3.9 | 0.064 | 0.005 |
| 2.0 | 0.043 | 0.001 |
| 1.0 | 0.039 | 0.000 |
| 0.5 | 0.040 | 0.004 |
| 0.2 | 0.040 | 0.002 |
| 0.0 | 0.038 | 0.002 |
| Blank | 0.038 | 0.002 |

**Tabelle 4C**

| Sandwich Immuno-Erythropoietinrezeptor-Bindungsassay | | |
|---|---|---|
| Festphasen gebundener Anti-human IgG monoklonaler Antikörper (5µg/ml) | | |
| Erythropoietinrezeptor-Fc-Fusionsprotein (100 ng/ml - 30 min/RT) | | |
| Die Detektion des gebundenen Erythropoietins erfolgte mittels Anti-EPO-Antikörpern gekoppelt an Meerettichperoxidase. | | |

| [EPO] in pg/ml | Monoklonaler Antikörper 89-113-107-Peroxidase-Konjugat (optische Dichte bei 492 nm) | |
|---|---|---|
| | Mittelwert | Standardab |
| 1000.0 | 1.986 | 0.012 |
| 500.0 | 1.374 | 0.016 |
| 250.0 | 0.670 | 0.026 |
| 125.0 | 0.277 | 0.015 |
| 62.5 | 0.124 | 0.013 |
| 31.2 | 0.069 | 0.004 |
| 15.6 | 0.049 | 0.003 |
| 7.8 | 0.037 | 0.003 |
| 3.9 | 0.028 | 0.001 |
| 2.0 | 0.024 | 0.002 |
| 1.0 | 0.022 | 0.001 |
| 0.5 | 0.023 | 0.001 |
| 0.2 | 0.020 | 0.001 |
| 0.0 | 0.022 | 0.003 |
| Blank | 0.021 | 0.001 |

**Tabelle 4D**

| [EPO] in pg/ml | Polyklonaler Kaninchenantikörper-Peroxidase-Konjugat 1095 (optische Dichte bei 492 nm) | |
|---|---|---|
| | Mittelwert | Standardabw. |
| 1000.0 | 3.303 | 0.027 |
| 500.0 | 2.446 | 0.127 |
| 250.0 | 1.264 | 0.024 |
| 125.0 | 0.662 | 0.022 |
| 62.5 | 0.354 | 0.016 |
| 31.2 | 0.213 | 0.010 |
| 15.6 | 0.148 | 0.010 |
| 7.8 | 0.113 | 0.003 |
| 3.9 | 0.090 | 0.007 |
| 2.0 | 0.082 | 0.009 |
| 1.0 | 0.072 | 0.003 |
| 0.5 | 0.067 | 0.001 |
| 0.2 | 0.065 | 0.002 |
| 0.0 | 0.065 | 0.004 |
| Blank | 0.067 | 0.004 |

**Tabelle 5**

| | | | | |
|---|---|---|---|---|
| Festphase: Anti-human IgG Antikörper | | | | |
| 1. Schritt: Anlagerung des EPO-Rezeptor-Fc-Fusionsprotein | | | | |
| 2. Schritt: Zugabe der Proben bzw. des EPO-Fc-Fusionsprotein | | | | |
| 3. Schritt: Zugabe eines Peroxidase-Konjugates eines monoklonalen Antikörpers (89-146-057), der die biologische Wirkung von EPO neutralisieren kann. | | | | |

| [EPO] ng/ml | EPO-Fc | | Glutaraldehyd-EPO-Dimer/Oligomer | |
|---|---|---|---|---|
| | Mittel | Stdabw | Mittel | Stdabw |
| 1000 | 4.138 | 0.145 | 0.029 | 0.001 |
| 333.3 | 1.731 | 0.064 | 0.028 | 0.002 |
| 111.1 | 0.625 | 0.024 | 0.027 | 0.002 |
| 37.0 | 0.247 | 0.010 | 0.023 | 0.002 |
| 12.3 | 0.092 | 0.004 | 0.024 | 0.001 |
| 4.1 | 0.048 | 0.003 | 0.022 | 0.002 |
| 1.4 | 0.035 | 0.003 | 0.025 | 0.002 |
| 0.5 | 0.028 | 0.003 | 0.024 | 0.003 |
| 0.0 | 0.024 | 0.002 | 0.023 | 0.002 |

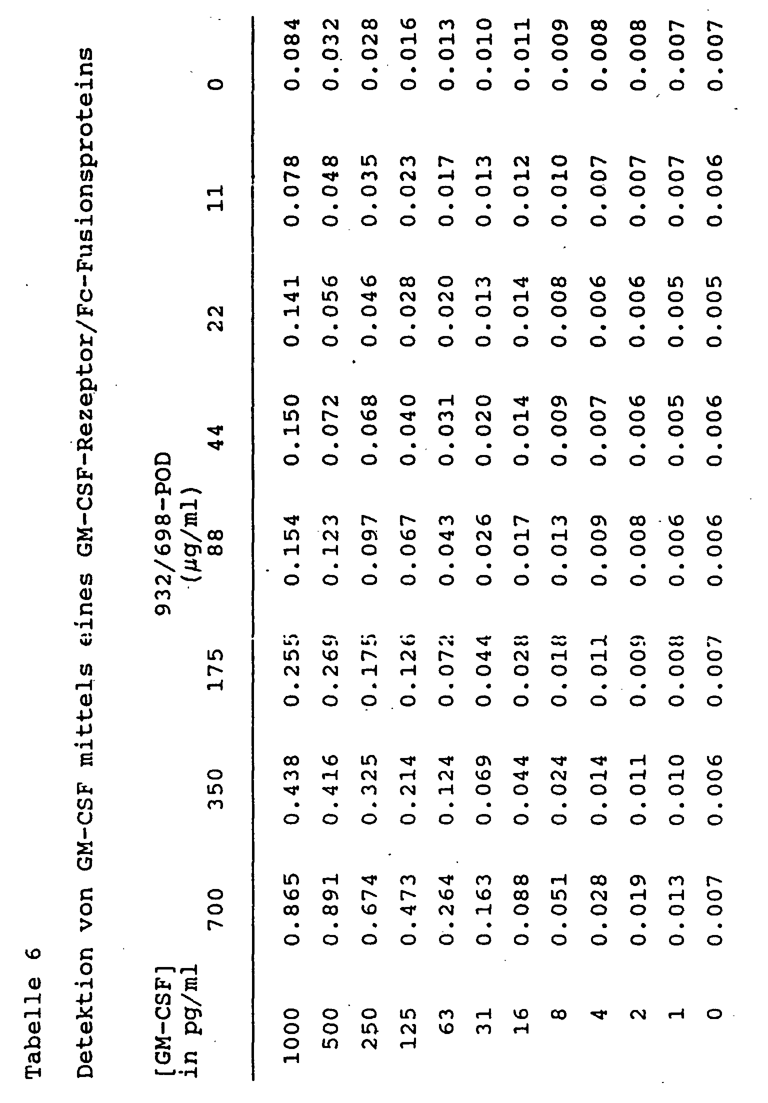

## Patentansprüche

1. Verfahren zum Nachweis und zur Bestimmung eines biologisch aktiven Mediators und/oder eines Derivats davon in einer Flüssigkeit, worin ein rekombinanter, löslicher Rezeptor des nachzuweisenden Mediators mit einer Probe, die den Mediator enthalten kann, in Kontakt gebracht und der an den Rezeptor gebundene Mediator durch einen für den Mediator spezifischen Antikörper direkt oder indirekt nachgewiesen wird, dadurch gekennzeichnet, daß der Mediator über die natürliche Rezeptorbindungsstelle an der Rezeptor gebunden wird.

2. Verfahren zum Nachweis und zur Bestimmung eines biologisch aktiven Mediators und/oder eines Derivats davon in einer Flüssigkeit, worin ein für den Mediator spezifischer Antikörper mit einer Probe, die den Mediator enthalten kann, in Kontakt gebracht und der an den Antikörper gebundene Mediator durch einen rekombinanten, löslichen Rezeptor des nachzuweisenden Mediators direkt oder indirekt nachgewiesen wird, dadurch gekennzeichnet, daß der Mediator über die natürliche Rezeptorbindungsstelle an der Rezeptor gebunden wird.

3. Verfahren nach Anspruch 1 oder 2, bei dem Dimere oder Multimere des Mediators oder Derivate davon bestimmt werden

4. Verfahren nach Anspruch 1, bei dem der Rezeptor an eine feste Phase gebunden wird.

5. Verfahren nach Anspruch 2, bei dem der für den Mediator spezifische Antikörper an eine feste Phase gebunden wird.

6. Verfahren nach Anspruch 1, worin der Rezeptor als rekombinantes Fusionsprotein, enthaltend den Fc-Teil eines Antikörpers vorliegt.

7. Verfahren nach Anspruch 6, bei dem der Rezeptor über Fc-spezifische Antikörper an die feste Phase gebunden wird, anschließend Probenmaterial mit dem korrespondierenden zu bestimmenden Mediator aufgetragen wird und der Mediator mit einem markierten Antikörper oder Antiserum mit Spezifität für den Mediator nachgewiesen wird.

8. Verwendung des Verfahrens nach mindestens einem der Ansprüche 1 bis 3 für ein Screening nach Agonisten oder Antagonisten des Mediators oder des Rezeptors.

9. Verwendung des Verfahrens nach mindestens einem der Ansprüche 1 bis 3 zur Bestimmung der Affinität zwischen Mediator und dessen Rezeptor.

10. Verwendung des Verfahrens nach mindestens einem der Ansprüche 1 bis 3 zur Identifikation und Analyse von modifizierten Mediatoren ("Muteine") oder Teilen der Mediatoren (z.B. Oligopeptide).

11. Verwendung des Verfahrens nach mindestens einem der Ansprüche 1 bis 3 zur Identifikation und Analyse von Substanzen, die die Interaktion von pathogenen Organismen (zum Beispiel Viren oder Bakterien) mit deren zellulären Rezeptoren beeinflussen.

12. Verwendung des Verfahrens nach mindestens einem der Ansprüche 1 bis 3 zur Identifikation von Substanzen, die die Interaktion von zellulären Adhäsionsmolekülen beeinflussen.

13. Verfahren nach mindestens einem der Ansprüche 1 bis 3, wobei der Rezeptor ein Cytokinrezeptor ist.

14. Verfahren nach mindestens einem der Ansprüche 1 bis 3, wobei der Rezeptor ein Hormonrezeptor ist.

15. Verfahren nach mindestens einem der Ansprüche 1 bis 3, wobei der Rezeptor ein Neurotransmitter-Rezeptor ist.

16. Verfahren nach mindestens einem der Ansprüche 1 bis 3, wobei der Rezeptor ein zellulärer Rezeptor für einen pathogenen Organismus, z.B. ein Virus ist.

17. Verfahren nach mindestens einem der Ansprüche 1 bis 3, wobei der Rezeptor der Interleukin-4-Rezeptor oder ein Derivat davon ist.

18. Verfahren nach mindestens einem der Ansprüche 1 bis 3, wobei der Rezeptor ein Erythropoietin-Rezeptor oder ein Derivat davon ist.

19. Verfahren nach mindestens einem der Ansprüche 1 bis 3, wobei der Rezeptor ein Interleukin 1-Rezeptor oder ein Derivat davon ist.

20. Verfahren nach mindestens einem der Ansprüche 1 bis 3, wobei der Rezeptor der Interleukin 7-Rezeptor oder ein Derivat davon ist.

21. Verfahren nach mindestens einem der Ansprüche 1 bis 3, wobei der Rezeptor der GM-CSF-Rezeptor oder ein Derivat davon ist.

22. Verfahren nach mindestens einem der Ansprüche 1 bis 3, wobei der Rezeptor der IL-8-Rezeptor oder ein Derivat davon ist.

23. Verfahren nach mindestens einem der Ansprüche 1 bis 3, wobei der Rezeptor ein TNF-Rezeptor oder ein Derivat davon ist.

24. Verfahren nach Anspruch 17 zum diagnostischen Nachweis von Interleukin-4 bei Erkrankungen mit verstärktem Auftreten von TH2-T-Zellen, zum Beispiel allergischen Erkrankungen und Infektionen.

## Claims

1. A method for detecting and determining a biologically active mediator and/or a derivative thereof in a fluid, wherein a recombinant, soluble receptor for the mediator to be detected is brought into contact with a sample, which can contain the mediator, and the mediator, which is bound to the receptor, is detected directly or indirectly by means of an antibody which is specific for the mediator, wherein the mediator is bound to the receptor by way of the natural receptor binding site.

2. A method for detecting and determining a biologically active mediator and/or a derivative thereof in a fluid, wherein an antibody, which is specific for the mediator, is brought into contact with a sample, which can contain the mediator, and the mediator, which is bound to the antibody, is detected directly or indirectly by means of a recombinant, soluble receptor for the mediator to be detected, wherein the mediator is bound to the receptor by way of the natural receptor binding site.

3. The method as claimed in claim 1 or 2, wherein dimers or multimers of the mediator, or derivatives thereof, are determined.

4. The method as claimed in claim 1, wherein the receptor is bound to a solid phase.

5. The method as claimed in claim 2, wherein the antibody which is specific for the mediator is bound to a solid phase.

6. The method as claimed in claim 1, wherein the receptor is present as a recombinant fusion protein containing the Fc moiety of an antibody.

7. The method as claimed in claim 6, wherein the receptor is bound to the solid phase via Fc-specific antibodies and, subsequently, sample material containing the corresponding mediator to be determined is applied, and the mediator is detected using a labeled antibody or antiserum having specificity for the mediator.

8. The use of the method as claimed in at least one of claims 1 to 3 for screening for agonists or antagonists of the mediator or of the receptor.

9. The use of the method as claimed in at least one of claims 1 to 3 for determining the affinity between a mediator and its receptor.

10. The use of the method as claimed in at least one of claims 1 to 3 for identifying and analyzing modified mediators ("muteins") or parts of the mediators (e.g. oligopeptides).

11. The use of the method as claimed in at least one of claims 1 to 3 for identifying and analyzing substances which influence the interaction of pathogenic organisms (for example viruses or bacteria) with their cellular receptors.

12. The use of the method as claimed in at least one of claims 1 to 3 for identifying substances which influence the interaction of cellular adhesion molecules.

13. The method as claimed in at least one of claims 1 to 3, wherein the receptor is a cytokine receptor.

14. The method as claimed in at least one of claims 1 to 3, wherein the receptor is a hormone receptor.

15. The method as claimed in at least one of claims 1 to 3, wherein the receptor is a neurotransmitter receptor.

16. The method as claimed in at least one of claims 1 to 3, wherein the receptor is a cellular receptor for a pathogenic organism, e.g. a virus.

17. The method as claimed in at least one of claims 1 to 3, wherein the receptor is the interleukin-4 receptor, or a derivative thereof.

18. The method as claimed in at least one of claims 1 to 3, wherein the receptor is an erythropoietin receptor, or a derivative thereof.

19. The method as claimed in at least one of claims 1 to 3, wherein the receptor is an interleukin-1 receptor, or a derivative thereof.

20. The method as claimed in at least one of claims 1 to 3, wherein the receptor is the interleukin-7 receptor, or a derivative thereof.

21. The method as claimed in at least one of claims 1 to 3, wherein the receptor is the GM-CSF receptor, or a derivative thereof.

22. The method as claimed in at least one of claims 1 to 3, wherein the receptor is the IL-8 receptor, or a derivative thereof.

23. The method as claimed in at least one of claims 1 to 3, wherein the receptor is a TNF receptor, or a derivative thereof.

24. The method as claimed in claim 17 for the diagnostic detection of interleukin-4 in diseases with an increased appearance of TH2 T-cells, for example allergic diseases and infections.

## Revendications

1. Procédé pour la détection et pour la détermination d'un médiateur biologiquement actif et/ou d'un dérivé de celui-ci dans un liquide, procédé dans lequel un récepteur soluble recombinant du médiateur à détecter est mis en contact avec un échantillon qui peut contenir le médiateur, et le médiateur fixé au récepteur est détecté directement ou indirectement au moyen d'un anticorps spécifique du médiateur, caractérisé en ce que le médiateur est fixé au récepteur par le site de liaison naturel du récepteur.

2. Procédé pour la détection et pour la détermination d'un médiateur biologiquement actif et/ou d'un dérivé de celui-ci dans un liquide, procédé dans lequel un anticorps spécifique du médiateur est mis en contact avec un échantillon qui peut contenir le médiateur, et le médiateur fixé à l'anticorps est détecté directement ou indirectement au moyen d'un récepteur soluble recombinant du médiateur à détecter, caractérisé en ce que médiateur est fixé au récepteur par le site de liaison naturel du récepteur.

3. Procédé selon la revendication 1 ou 2, dans lequel les dimères ou multimères du médiateur ou des dérivés de ceux-ci sont déterminés.

4. Procédé selon la revendication 1, dans lequel le récepteur est fixé à une phase solide.

5. Procédé selon la revendication 2, dans lequel l'anticorps spécifique du médiateur est fixé à une phase solide.

6. Procédé selon la revendication 1, dans lequel le récepteur est présent sous forme d'une protéine de fusion recombinante contenant le segment Fc d'un anticorps.

7. Procédé selon la revendication 6, dans lequel le récepteur est fixé à la phase solide par l'intermédiaire d'anticorps spécifique de Fc, une substance échantillon avec le médiateur à déterminer correspondant est ensuite appliquée, et le médiateur est détecté à l'aide d'un antisérum ou d'un anticorps marqué, à spécificité pour le médiateur.

8. Utilisation du procédé selon au moins l'une des revendications 1 à 3, pour un criblage à la recherche d'agonistes ou d'antagonistes du médiateur ou du récepteur.

9. Utilisation du procédé selon au moins l'une des revendications 1 à 3, pour la détermination de l'affinité entre un médiateur et son récepteur.

10. Utilisation du procédé selon au moins l'une des revendications 1 à 3, pour l'identification et l'analyse de médiateurs modifiés ("mutéines") ou de parties des médiateurs (par exemple des oligo-peptides).

11. Utilisation du procédé selon au moins l'une des revendications 1 à 3, pour l'identification et l'analyse de substances qui influencent l'interaction d'organismes pathogènes (par exemple des virus ou des bactéries) avec leurs récepteurs cellulaires.

12. Utilisation du procédé selon au moins l'une des revendications 1 à 3, pour l'identification de substances qui influent sur l'interaction de molécules d'adhérence cellulaire.

13. Procédé selon au moins l'une des revendications 1 à 3, dans lequel le récepteur est un récepteur de cytokine.

14. Procédé selon au moins l'une des revendications 1 à 3, dans lequel le récepteur est un récepteur hormonal.

15. Procédé selon au moins l'une des revendications 1 à 3, dans lequel le récepteur est un récepteur de neurotransmetteur.

16. Procédé selon au moins l'une des revendications 1 à 3, dans lequel le récepteur est un récepteur cellulaire pour un organisme pathogène, par exemple un virus.

17. Procédé selon au moins l'une des revendications 1 à 3, dans lequel le récepteur est le récepteur d'interleukine 4 ou un dérivé de celui-ci.

18. Procédé selon au moins l'une des revendications 1 à 3, dans lequel le récepteur est un récepteur d'érythropoïétine ou un dérivé de celui-ci.

19. Procédé selon au moins l'une des revendications 1 à 3, dans lequel le récepteur est le récepteur d'interleukine 1 ou un dérivé de celui-ci.

20. Procédé selon au moins l'une des revendications 1 à 3, dans lequel le récepteur est le récepteur d'interleukine 7 ou un dérivé de celui-ci.

21. Procédé selon au moins l'une des revendications 1 à 3, dans lequel le récepteur est le récepteur de GM-CSF ou un dérivé de celui-ci.

22. Procédé selon au moins l'une des revendications 1 à 3, dans lequel le récepteur est le récepteur d'IL-8 ou un dérivé de celui-ci.

23. Procédé selon au moins l'une des revendications 1 à 3, dans lequel le récepteur est un récepteur de TNF ou un dérivé de celui-ci.

24. Procédé selon la revendication 17, pour la détection de diagnostic d'interleukine 4 dans des maladies à apparition accentuée de lymphocytes T TH2, par exemple de maladies allergiques et d'infections.
